# EUROPEAN PATENT APPLICATION

(11) **EP 4 600 254 A1**
(43) Date of publication of application: **13.08.2025**
(21) Application number: 23875251.3
(22) Date of filing: 06.10.2023
(51) Int. Cl.: C07K 14/005, C12N 15/86, A61P 11/00, A61K 48/00

(54) **ADENO-ASSOCIATED VIRUS VECTOR CAPABLE OF SPECIFIC GENE DELIVERY TO PULMONARY BRONCHI**

(30) Priority: 06.10.2022 KR 20220127751
(71) Applicant: Glugenetherapeutics Inc., Daejeon 34028 (KR)
(72) Inventor: JANG, Jae-Hyung, Seoul 07997 (KR); KIM, Yoojin, Seoul 06625 (KR)
(74) Representative: Schiweck Weinzierl Koch Patentanwälte Partnerschaft mbB
(86) International application number: PCT/KR2023/015391
(87) International publication number: WO 2024/076190

(57) **Abstract**

The present invention provides a mutant of adeno-associated virus serotype 1 (AAV1) capsid protein, a nucleic acid encoding the mutant of the AAV1 capsid protein, a recombinant AAV1 vector carrying the nucleic acid encoding the mutant of AA1 capsid protein, and a pharmaceutical composition containing the vector, wherein the mutant has a specific amino acid substituted at a certain position, compared to the amino acid sequence of the wild type AAV capsid protein. Specifically, the recombinant virus vector carrying the nucleic acid encoding the mutant of the AAV1 capsid protein enhances the expression of introduced genes in the pulmonary bronchi when delivered in an aerosol state through the bronchi, making it useful for the prevention or treatment of diseases related to the pulmonary bronchi.

## Description

### [Technical Field]

The present invention relates to a mutant of an adeno-associated virus (AAV) capsid protein, which is particularly useful for the expression of introduced genes in the pulmonary bronchi when recombinant viral vectors containing mutants of the AAV1 capsid protein are delivered in an aerosol state through the bronchi.

### [Background Art]

In order to effectively perform gene therapy, the development of gene delivery technology that can deliver therapeutic genes to the desired target cells and achieve high expression efficiency is of the utmost priority.

Among these gene delivery technologies, AAVs are non-pathogenic viruses that have no side effects on infected cells that have infiltrated and a low probability of causing mutations in the genetic information of target cells, making it superior in terms of safety compared to other gene therapy technologies.

Adeno-associated viruses (AAVs) are non-enveloped single-stranded DNA viruses that can infect both dividing and non-dividing cells. AAVs can replicate only in the presence of a helper virus and are non-pathogenic to humans. With these characteristics, an AAV is a useful method of introducing genes into various cells and is used as a useful vector for gene therapy.

It is known that AAVs have various serotypes and that the characteristics of a host or virus differ depending on the serotype. Serotype 2 (AAV2) is a serotype that has been widely studied for a long time and can infect various cells. Serotype 1 (AAV1), Serotype 5 (AAV5), and Serotype 6 (AAV6) are serotypes with more tissue infection specificity. It is known that AAV1 has high gene introduction efficiency with respect to muscles, the liver, the trachea, and the central nervous system, AAV5 high gene introduction efficiency with respect to the central nervous system, the liver, and the retina, and AAV6 high gene introduction efficiency with respect to the heart, muscles, and the liver. Although the characteristics of gene delivery to specific tissues vary depending on the serotype, it is still easy to transfer to other tissues, so it can be said that the development of new AAV vectors that can improve safety and efficiency by enhancing tissue specificity is essential.

Meanwhile, bronchitis and bronchiectasis are major diseases that occur in the bronchi of the lungs. In particular, bronchiectasis can cause complications such as hemoptysis (a symptom of coughing up blood or sputum mixed with blood), persistent dyspnea, and respiratory failure, so it needs to be quickly diagnosed and treated. Hemoptysis is a potentially life-threatening complication of bronchiectasis, and bronchial artery embolization or surgical treatment may be considered to control hemoptysis.

Although attempts have been made to improve gene transfer efficiency by modifying the AAV capsid protein, no studies have yet been reported on AAV targeting the pulmonary bronchi.

### [Related Art Documents]

### [Patent Documents]

International Publication No. WO/2017/201121 (November 23, 2017)
Japanese Patent Laid-open Publication No. 2021-0010372 (February 04, 2021)

### [Disclosure]

### [Technical Problem]

Therefore, the inventors of the present invention have made great efforts to solve the above problems and have completed the present invention by developing a protein variant based on the capsid of a novel adeno-associated virus (AAV) serotype 1 targeting the pulmonary bronchi, which has the potential to deliver a gene therapeutic agent solving the fundamental genetic cause of pulmonary bronchial diseases such as bronchitis or bronchiectasis.

Accordingly, the present invention is directed to providing a mutant of the AAV1 capsid protein for improving the efficiency of gene transfer into target tissue or cells and/or the efficiency of genetic information expression by recombinant AAV.

The present invention is also directed to providing a nucleic acid encoding the mutant of the AAV1 capsid protein.

The present invention is also directed to providing a recombinant AAV1 vector which includes a nucleic acid encoding the mutant of the AAV1 capsid protein.

The present invention is also directed to providing a pharmaceutical composition including the recombinant AAV1 vector.

The present invention is also directed to providing a gene delivery vehicle including the recombinant AAV1 vector.

### [Technical Solution]

Hereinafter, the present invention will be described in detail below.

The present invention relates to a mutant of an adeno-associated virus serotype 1 (AAV1) capsid protein.

The term "adeno-associated virus" or "AAV" used herein refers to all adeno-associated viruses used in gene therapy, including derivatives, virus subtypes and naturally-occurring and recombinant forms thereof. Various serotypes of AAV are capable of being used as recombinant gene delivery viruses for transducing different cell types. The genomic sequences of various serotypes of AAV, as well as the sequences of native terminal repeats (TRs), Rep proteins and capsid subunits are known in the art. These sequences can be found in the literature or in public databases such as GenBank. For example, GenBank Accession Nos. NC_002077(AAV-1) and AF063497(AAV-1) may be referenced.

The term "serotype" used herein may be identified by a serological or DNA sequencing method, and refers to a subset of AAV that can be distinguished by its antigenic properties.

The term "capsid" used herein is a protein encoded by a cap gene present in a viral genome, and refers to a protein constituting the outer shell of a virus. A wild-type AAV genome or cap gene encodes three types of capsid proteins (VP1, VP2, and VP3). The wild-type AAV1 capsid protein includes an amino acid sequence represented by SEQ ID NO: 1.

In one embodiment, the present invention provides a mutant of the adeno-associated virus serotype 1 (AAV1) capsid protein, wherein the mutant is a mutant of the AAV1 capsid protein in which an amino acid present at one or more of positions 326, 452, and 456 in the amino acid sequence of SEQ ID NO: 1 of the wild-type AAV1 capsid protein is substituted.

In one embodiment, the present invention provides a mutant of the AAV1 capsid protein, wherein the mutant includes a mutant of the AAV1 capsid protein in which the threonine at position 326 is substituted with alanine and glutamine at position 452 is substituted with proline in the amino acid sequence of SEQ ID NO: 1 of the wild-type AAV1 capsid protein.

In one embodiment, the present invention is a mutant of the AAV1 capsid protein, wherein the mutant includes a mutant of the AAV1 capsid protein in which alanine at position 456 in the amino acid sequence of SEQ ID NO: 1 of the wild-type AAV1 capsid protein is substituted with threonine.

In one embodiment, the mutant of the AAV1 capsid protein according to the present invention includes or consists of an amino acid represented by SEQ ID NO: 2 or 3.

In one embodiment of the present invention, the mutant of the AAV1 capsid protein represented by SEQ ID NO: 2 was named #3-32, and the mutant of the AAV1 capsid protein represented by SEQ ID NO: 3 was named #3-65.

The term "wild type" used herein refers to a type that can be most commonly found in wild populations. For mutant types, the wild type refers to the phenotype or individual considered to be basic. The wild-type is also called a "normal type." Meanwhile, the "mutant" used herein means a protein, virus, cell, or individual that is generated by the characteristic changes in mutated genes. In addition, the "mutant" used herein may also refer to a gene itself, which causes the mutation.

In one embodiment, the present invention includes a nucleic acid encoding a mutant of the AAV1 capsid protein. The nucleic acid of the present invention encodes the mutant of the AAV1 capsid protein. The nucleic acid of the present invention is prepared by substituting at least one base in the base sequence of the nucleic acid (*cap* gene) encoding the AAV1 capsid protein with another base. The nucleic acid of the present invention may be present in the form of DNA, but may also be in the form of RNA or a chimera of DNA and RNA. In addition, the nucleic acid of the present invention also includes a complementary nucleic acid (e.g., cDNA). The nucleic acid of the present invention may be single-stranded, or double-stranded, and preferably, double-stranded.

The present invention provides a nucleic acid encoding a mutant of the AAV1 capsid protein consisting of an amino acid sequence represented by SEQ ID NO: 2 or 3, and although not particularly limited, as one embodiment, a nucleic acid having a base sequence represented by SEQ ID NO: 4 or 5 is exemplified.

The nucleic acid of the present invention may be operably linked to a suitable control sequence. Control sequences include promoter sequences, polyadenylation signals, transcription termination sequences, upstream regulatory domains, internal ribosome entry sites (IRES), and enhancers. Promoter sequences include an inducible promoter sequence and a constitutive promoter sequence. Control sequences may be unique to the AAV from which a capsid protein is derived, or may be foreign, natural or synthetic. The present invention also includes recombinant DNA capable of expressing a mutant of the AAV1 capsid protein, including the nucleic acid of the present invention.

The recombinant DNA is useful for delivering the nucleic acid of the present invention to cells *in vitro*, *ex vivo*, and *in vivo,* and imparting the ability to express a mutant of an AAV1 capsid protein to the corresponding cells. In addition, the cells to which the nucleic acid of the present invention is delivered are useful for producing recombinant AAV particles. The recombinant DNA may be used to deliver or introduce the nucleic acid of the present invention especially to eukaryotic cells, preferably, animal cells, and more preferably, mammalian cells.

In the present invention, recombinant DNA may be prepared by harboring the nucleic acid of the present invention in DNA that is used as a vector. For example, as recombinant DNA, a plasmid, a phage, a transposon, a cosmid, episomal DNA, or a viral genome may be used.

For example, by harboring the nucleic acid (*cap* gene) encoding the mutant of the AAV1 capsid protein of the present invention in a plasmid, a packaging plasmid may be produced. The packaging plasmid may further include any nucleic acid sequence, such as a nucleic acid (*rep* gene) encoding a replicase (Rep) protein. Preferably, the *rep* gene includes AAV2-derived Rep.

In the nucleic acid sequence of the *cap* gene carried in a known packaging plasmid, even by substituting at least one base in a PLA2 domain-coding region with another base, recombinant DNA including the nucleic acid of the present invention may be prepared. There is no particular limitation on the packaging plasmid, but examples include a packaging plasmid carrying the *cap* gene, and preferably, a packaging plasmid carrying the *cap* gene and *rep* gene. In one example, in the present invention, a recombinant AAV1 vector p #3-32 or p #3-65, represented by SEQ ID NO: 6 or 7, as a packaging plasmid carrying the nucleic acid (*cap* gene) encoding the mutant of the AAV1 capsid protein of the present invention and the *rep* gene, was constructed.

A method of introducing a base substitution into the nucleic acid may be carried out by a known method, and may be accomplished, but is not particularly limited to, by performing PCR using a commercially available reagent, e.g., a mutagenesis basal kit (TAKARA BIO INC.) according to the instructions contained in the kit.

Accordingly, the present invention provides a recombinant AAV1 vector, which includes a nucleic acid encoding the AAV1 capsid protein variant.

The recombinant AAV vector of the present invention is useful for gene introduction into target cells. A gene introduced into the recombinant AAV vector of the present invention is strongly expressed in the target cells.

The "AAV vector" used herein refers to any vector that includes or is derived from a component of an adeno-associated virus (AAV) and is suitable for infecting any mammalian cell including human cells of many tissue types, such as the brain, heart, lung, skeletal muscle, liver, kidney, spleen or pancreas, whether *in vitro* or *in vivo.* The term "AAV vector" may be used to refer to an AAV-type viral particle (or virion) including at least a nucleic acid molecule encoding a protein of interest.

The "AAV virus," "AAV viral particle," or "rAAV vector particle" used herein refers to a viral particle consisting of at least one AAV capsid protein (e.g., any capsid protein of wild-type AAV) and a polynucleotide rAAV vector encapsidated in a capsid. When a particle includes a heterologous polynucleotide (i.e., a polynucleotide other than a wild-type AAV genome, such as a transgene delivered to mammalian cells), it is typically referred to as an "rAAV vector particle" or simply an "rAAV vector." Accordingly, the generation of the rAAV particle must include rAAV generation, which is because such a vector is contained in the rAAV particle.

"Packaging" refers to a series of intracellular events that result in the assembly and encapsidation of AAV particles.

AAV "rep" and "cap" genes refer to polynucleotide sequences that encode AAV replication and encapsidation proteins. AAV rep and cap are referred to as AAV "packaging genes" in this specification.

"Helper virus" for AAV refers to a virus that allows AAV (e.g., wild-type AAV) to be replicated and packaged by mammalian cells. A variety of helper viruses for AAV, for example, adenoviruses, herpes viruses, and poxviruses such as vaccinia, are known in the art. While subgroup C adenovirus serotype 5 is most frequently used, adenoviruses include several different subgroups. Numerous adenoviruses derived from humans, non-human mammals, and avians are known, and available from depositories such as ATCC. Herpes viruses include, for example, not only herpes simplex virus (HSV) and Epstein-Barr virus (EBV), but also cytomegalovirus (CMV) and pseudorabies virus (PRV); and these are also available from depositories such as ATCC.

"Helper virus function(s)" refer to function(s) encoded in the helper virus genome that allows AAV replication and packaging (along with other requirements for replication and packaging described herein). As described herein, "helper virus function" may be provided in various ways that include providing a helper virus, or for example, providing a polynucleotide sequence encoding essential function(s) to producer cells in trans. For example, a plasmid or another expression vector, which includes a nucleotide sequence encoding one or more adenovirus proteins, is transfected into producer cells along with an rAAV vector.

In one embodiment, the AAV1 vector according to the present invention has an improved transduction profile for target tissue as compared to an AAV1 vector including a wild-type capsid protein. In other words, the AAV1 vector according to the present invention has obvious tissue targeting ability (e.g., tissue tropism).

The term "tropism" used herein means the specificity of an AAV capsid protein present in an AAV virus particle for infecting or transducing a specific type of cell or tissue.

The tropism of an AAV capsid for a specific type of cell or tissue may be determined by measuring the ability of AAV vector particles to transfect or transduce a specific type of cells or tissue due to the AAV1 capsid protein therein using a standard analysis method well known in the art, which is the same as described in the examples of the present specification.

That is, "tropism" refers to the ability of an AAV vector or virion to infect one or more specific cell types. However, it can also include whether a vector serves to transduce one or more specific cell types. That is, tropism refers to the expression (e.g., transcription and in some cases, translation) of a sequence delivered by an AAV vector or virion, depending on the case and preferably, in cells, for example, the expression of heterologous nucleotide sequence(s) in the case of a recombinant virus, following the preferential introduction of an AAV vector or virion into specific cell or tissue type(s) and/or the preferential interaction with a cell surface facilitating entry into a specific cell or tissue type.

The term "transduction" used herein refers to the ability of an AAV vector or virion to infect one or more specific cell types. That is, transduction means that an AAV vector or virion is introduced into cells to deliver a genetic material contained in the AAV vector or virion to cells, thereby achieving expression from the vector genome. In some, but not all, cases, transduction and tropism may be correlated.

The AAV described in the present invention includes amino acid modification(s) in one or more capsid proteins, which imparts a new or enhanced tissue tropism property. The AAV1 variant according to the present invention targets the pulmonary bronchi.

The term "pulmonary bronchi tropism" used in the specification refers to tropism for the pulmonary bronchi.

In some embodiments, the pulmonary bronchi tropism of a peptide-modified hybrid AAV capsid protein is further increased at least 5%, 10%, 20%, 30%, 40%, or 50% or more, compared to the pulmonary bronchi tropism of a wild-type AAV capsid protein without the peptide.

In addition, the present invention may include a pharmaceutical composition including the recombinant AAV1 vector. The composition may further include a pharmaceutically acceptable carrier.

The "pharmaceutically acceptable carrier" includes any material that allows a component to retain biological activity without causing adverse physiological reactions such as unintended immune reactions when combined with an active ingredient of the composition. Pharmaceutically acceptable carriers include water, phosphate buffered saline, emulsions such as an oil/water emulsion, and wetting agents. Compositions including these carriers are formulated by well known conventional methods such as those described in Remington's Pharmaceutical Sciences, current Ed., Mack Publishing Co., Easton Pa. 18042, USA; A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., 7th ed., Lippincott, Williams, & Wilkins; and Handbook of Pharmaceutical Excipients (2000) A. H. Kibbe et al., 3rd ed. Amer. Pharmaceutical Assoc.

In one embodiment, the pharmaceutical composition according to the present invention may be a pharmaceutical composition for preventing or treating pulmonary bronchial diseases, specifically, bronchitis or bronchiectasis.

The term "treatment" used herein refers to the administration of an active agent in any type of intervention or process performed on a subject to reverse, alleviate, improve, inhibit, delay or prevent the progression, development, severity or relapse of a disease-related syndrome, complication, symptom, or biochemical sign. The treatment may be performed on a subject with a disease or a subject without a disease (e.g., for prevention).

In addition, in one embodiment, the present invention includes a method of preventing or treating pulmonary bronchial diseases, specifically, bronchitis or bronchiectasis, which includes administering a therapeutically effective amount of the pharmaceutical composition to a subject.

The "administration" used herein refers to the physical introduction of a therapeutic agent or a composition including a therapeutic agent to a subject using any one of various methods and delivery systems known by those of ordinary skill in the art. Preferable administration routes include parenteral administration routes such as intravenous, intraperitoneal, intramuscular, subcutaneous, intrathecal, intravitreal administration routes, for example, by injection or infusion. The phrase "parenteral administration" used herein generally refers to an administration method, other than intestinal and local administration by injection, which may be, but is not limited to, intravenous, intraperitoneal, intramuscular, intraarterial, intrathecal, intralymphatic, intralesional, intracapsular, intraorbital, intracardiac, intradermal, transtracheal, subcutaneous, subepidermal, intravitreal, intraarticular, subcapsular, subarachnoidal, intraspinal, epidural, or intrasternal injection or infusion, or *in vivo* electroporation. In a specific embodiment, it was confirmed that the AAV vector of the present invention is capable of bronchi-specific gene delivery by administering it in the form of an aerosol into the respiratory tract in an animal experiment using pigs.

The term "therapeutically effective amount" used herein refers to an amount of a drug that is effective in "treating" a disease or disorder in a subject, or reducing the risk, potential, possibility or occurrence of a disease or disorder (e.g., pulmonary arterial hypertension) alone or in combination with a different therapeutic agent. The "therapeutically effective amount" includes the amount of a drug or therapeutic agent that provides some improvement or benefit to a subject having a disease or disorder (e.g., the pulmonary hypertension disclosed herein) or the risk of a disease or disorder. Accordingly, the "therapeutically effective amount" is an amount that reduces the risk, potential, possibility or occurrence of a disease or disorder, or provides some alleviation or reduction, and/or reduces at least one indicator (e.g., a pulmonary bronchial disease) , and/or reduces at least one clinical symptom of a disease or disorder.

The term "subject" used herein includes a human or a non-human animal. The term "non-human animal" includes all vertebrates, including mammals, non-human primates, sheep, dog, cattle, chickens, and non-mammals such as amphibians and reptiles.

In addition, the present invention may include a drug delivery vehicle including the recombinant AAV1 vector. The composition may further include a known pharmaceutical acceptable carrier for use in the above-described applications.

The AAV1 vector of the present invention exhibits tropism for the pulmonary bronchi and has the ability to express genes specifically in the pulmonary bronchi, so it can be used as a vehicle to deliver drugs to the pulmonary bronchi. Specifically, it may be used as an AAV1 vaccine for infectious materials that infect the respiratory tract. While existing vaccines that are injected intravenously have the disadvantage of spreading throughout the body, which reduces the possibility of antibody production in the lung or airway areas, when a gene is delivered using the vector of the present invention, gene expression efficiency can be increased near the respiratory tract where infectious materials first come into contact, thereby increasing mucosal immunity compared to existing vaccines.

### [Advantageous Effects]

The present invention relates to a recombinant AAV1 capsid variant having high efficiency gene delivery ability by specifically targeting the pulmonary bronchi, and a recombinant viral vector including a nucleic acid encoding a mutant of the AAV1 capsid protein is useful for the expression of introduced genes in pulmonary bronchi-targeting cells when delivered in an aerosol state through the bronchi, thereby enabling the prevention or treatment of pulmonary bronchial diseases.

### [Description of Drawings]

FIG. 1 shows the packaging efficiency of wild-type AAV1 and recombinant AAV1 vectors of the present invention (#3-32 and #3-65 variants) as compared by genomic titer through quantitative PCR analysis.
FIG. 2 shows the analysis of the improvement in HEK293T transduction efficiency by the proportion of cells expressing GFP among the total cultured cells.
FIG. 3 shows the results of efficient local delivery in lung tissue in which LacZ expression has been confirmed by staining the lungs extracted from 8-week-old C57BL/6 male mice with whole-mount β-galactosidase in order to confirm the pulmonary bronchial specificity of recombinant AAV1 vectors (#3-32(a) and #3-65(b) variants).
FIG. 4 shows LacZ expression in the pulmonary bronchi by the recombinant AAV1 vectors (#3-32(a) and #3-65(b) variants) through eosin / LacZ staining in order to confirm pulmonary bronchial specificity.
FIGS. 5A and 5B show the cleavage maps of the AAV1 vectors (#3-32 and #3-65 variants), respectively.
FIG. 5C shows the cleavage map of a pHelper plasmid.
FIG. 5D shows the cleavage map of a pCMV GFP plasmid.
FIG. 5E shows the cleavage map of a pCMV LacZ plasmid.
FIG. 6 shows the results of injecting a recombinant AAV1 vector (#3-65 variant) through the porcine airway in order to confirm pulmonary bronchial specificity.

### [Modes of the Invention]

Hereinafter, the present invention will be described in further detail with reference to examples according to the present invention, but the scope of the present invention is not limited by the examples presented below.

### [Examples]

### Example 1: Selection and construction of AAV1 capsid protein variant

### 1) Selection of AAV1 capsid protein variant

A plasmid pool was produced by inducing random point mutations in the *cap* gene of each of wild-type AAV variants (AAV1, AAV2, AAV4, AAV6, AAV8, and AAV9) using error-prone PCR and inserting random 7mer/9mer into the 3-fold protrusion of each serotype. After forming a calcium-phosphate complex with 7 to 70 ng of the AAV plasmid library, 25 µg of pBluescript, and 25 µg of pHelper, the calcium-phosphate complex was transfected into AAV293 cells to perform AAV packaging, and an AAV library pool carrying the *cap* gene information of each variant was produced.

After inhalation anesthesia of 8-week-old C57BL/6 male mice with isoflurane, an approximately 1-cm incision was made in the skin of the tracheal area. 1x10¹¹ vg/100 µL of the AAV library pool in PBS was loaded in a PenWu micro-aerosolizer (BioJane, Shanghai, China) (length of intratracheal portion: 1.25", outer diameter: 700 µm, inner diameter: 430 µm), and intratracheal injection was performed while confirming the passage of the needle through the airway.

After one week, 0.9% saline was perfused through the heart, and the lungs were removed. After homogenization, DNA was extracted from the entire lungs using a DNA mini kit (Qiagen), the *cap* gene of AAV variants showing a tropism for the entire lungs was amplified using the AAV *cap* gene-specific forward primer 5'-GCGGAAGCTTCGATCAACTACG-3' (SEQ ID NO: 8) and the reverse primer 5'-CGCAGAGACCAAAGTTCAACTGA-3' (SEQ ID NO: 9). A pulmonary tropic AAV library pool was produced with the genes to perform intratracheal injection in the same manner as above. After one week, perfusion and lung extraction were performed, and after chopping the lungs into small pieces for 30 seconds, cell dissociation was performed using collagenase II. Here, DNase I was added to prevent cell clustering caused by chromosomal DNA released from dead cells, resulting in minimized cell loss. After incubation at 37 °C for 4 to 6 hours, the total lung population in the form of single cells was obtained through pipetting, red blood cell lysis was performed at room temperature in a light-blocking state, and then the cells were transferred to FACS buffer through a cell strainer with a pore size of 70 µm. Afterward, to select AAV variants showing local tropism in lung tissue, 10 µg of α-sma-APC antibody (anti-alpha smooth muscle actin antibody) was added, followed by incubation at 4 °C. Subsequently, APC positive cells were sorted to select bronchial-related cells, and after cell lysis and DNA extraction, the *cap* gene of the AAV variants entering cells was amplified using the above AAV *cap* gene-specific primer information. This was produced into a secondary AAV library and the intratracheal injection and sorting operations were performed in the same manner. After three rounds of *in vivo* selection, the finally amplified *cap* gene was subcloned into a pSub2 plasmid through HindIII/NotI restriction and ligation, including the HindIII and NotI sequences at both ends, and electroporated into DH10β. Then the plasmid was purified (Qiagen Plasmid Maxi Kit) and stored in the form of a bronchi-tropic plasmid pool (pSub2 is a plasmid produced in the David Schaffer Lab (UC Berkeley) based on pSub201(ATCC) to subclone the *cap* gene using HindIII and NotI, Reference 1. Narendra Maheshri et al., Nature Biotechnology, 2006; 2. James T Koerber, Nature Protocols, 2006).

### 2) Construction of recombinant AAV1 vectors (AAV1 #3-32 and #3-65 variants)

### ① Construction of packaging plasmid mutants

The construction of multiple bronchi-specific plasmid mutants for loading a reporter gene was completed by subcloning multiple bronchi-tropic *cap* genes into a blood vessel-tropic plasmid pool through HindIII and NotI restriction in pXX2 (UC Berkeley, David Schaffer Lab) into which HindIII and NotI were introduced for the *cap* gene insertion.

### ② Plasmid introduction into AAV293 cells

### Construction of AAV1 #3-32 and #3-65 variants

A calcium-phosphate complex formed of 17 µg of a mutant plasmid, 17 µg of an ITR flanked reporter gene (pCMV-GFP, pCMV-LacZ, or pCMV-FGF12-IRES-GFP), and 17 µg of pHelper was transfected into AAV293 cells, and after approximately 48 hours, only a cell pellet was collected and then intracellular AAV was extracted therefrom through freezing-thawing. Afterward, cell debris was removed through centrifugation, and 10 U/mL of benzonase was incubated at 37 °C for 30 minutes to remove a nucleic acid derived from virus-producing cells.

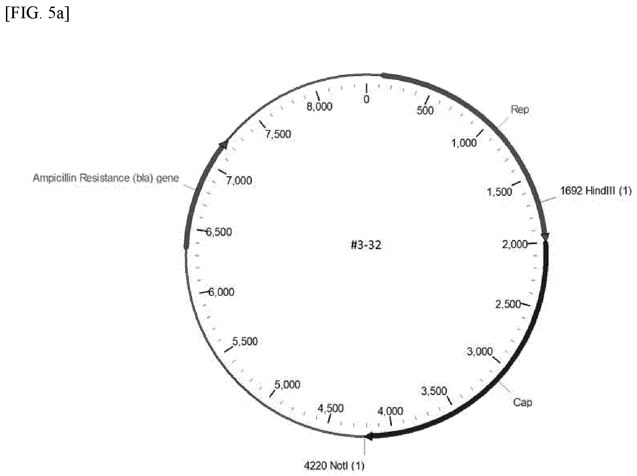

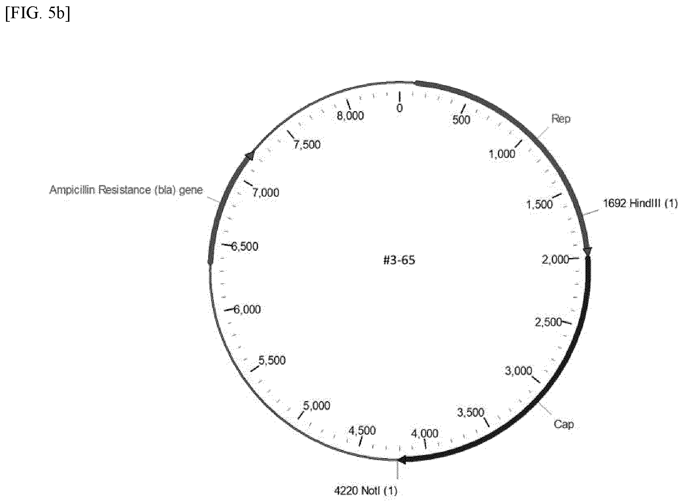

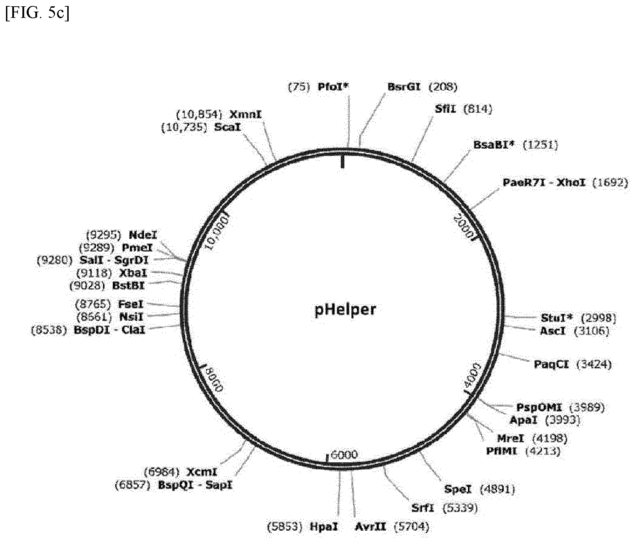

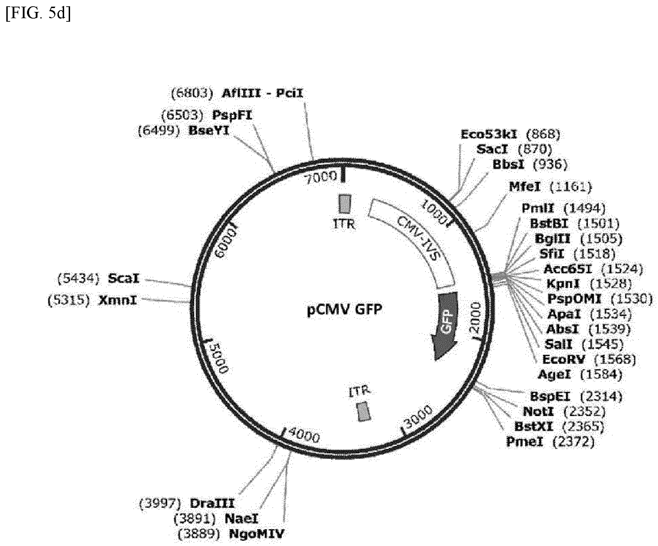

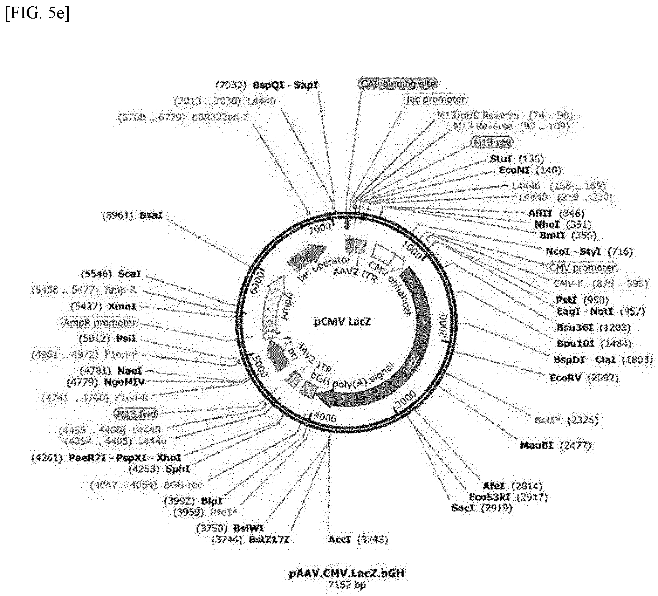

The cleavage maps of the constructed AAV1 #3-32 and #3-65 variants are shown in FIGS. 5A and 5B, and the entire base sequences of the AAV1 #3-32 and #3-65 variants are shown below.
**AAV-2 Rep gene** #3-32 Cap gene *Ampicillin Resistance (bla) gene(SEQ* ID NO: 6) 
GCGCGCCGATATCGTTAACGCCCCGCGCCGGCCGCTCTAGAACTAGTGGA
TCCCCCGGAAGATCAGAAGTTCCTATTCCGAAGTTCCTATTCTCTAGAAAGTATA
GGAACTTCTGATCTGCGCAGCCGCCATGCCGGGGTTTTACGAGATTGTGATTA
AGGTCCCCAGCGACCTTGACGGGCATCTGCCCGGCATTTCTGACAGCTTTGT
GAACTGGGTGGCCGAGAAGGAATGGGAGTTGCCGCCAGATTCTGACATGGA
TCTGAATCTGATTGAGCAGGCACCCCTGACCGTGGCCGAGAAGCTGCAGCG
CGACTTTCTGACGGAATGGCGCCGTGTGAGTAAGGCCCCGGAGGCCCTTTT
CTTTGTGCAATTTGAGAAGGGAGAGAGCTACTTCCACATGCACGTGCTCGTG
GAAACCACCGGGGTGAAATCCATGGTTTTGGGACGTTTCCTGAGTCAGATTC
GCGAAAAACTGATTCAGAGAATTTACCGCGGGATCGAGCCGACTTTGCCAAA
CTGGTTCGCGGTCACAAAGACCAGAAATGGCGCCGGAGGCGGGAACAAGGT
GGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATC
TCACGGAGCGTAAACGGTTGGTGGCGCAGCATCTGACGCACGTGTCGCAGA
CGCAGGAGCAGAACAAAGAGAATCAGAATCCCAATTCTGATGCGCCGGTGA
TCAGATCAAAAACTTCAGCCAGGTACATGGAGCTGGTCGGGTGGCTCGTGG
ACAAGGGGATTACCTCGGAGAAGCAGTGGATCCAGGAGGACCAGGCCTCAT
ACATCTCCTTCAATGCGGCCTCCAACTCGCGGTCCCAAATCAAGGCTGCCTT
GGACAATGCGGGAAAGATTATGAGCCTGACTAAAACCGCCCCCGACTACCT
 
GGTGGGCCAGCAGCCCGTGGAGGACATTTCCAGCAATCGGATTTATAAAATT
TTGGAACTAAACGGGTACGATCCCCAATATGCGGCTTCCGTCTTTCTGGGAT
GGGCCACGAAAAAGTTCGGCAAGAGGAACACCATCTGGCTGTTTGGGCCTG
CAACTACCGGGAAGACCAACATCGCGGAGGCCATAGCCCACACTGTGCCCT
TCTACGGGTGCGTAAACTGGACCAATGAGAACTTTCCCTTCAACGACTGTGT
CGACAAGATGGTGATCTGGTGGGAGGAGGGGAAGATGACCGCCAAGGTCGT
GGAGTCGGCCAAAGCCATTCTCGGAGGAAGCAAGGTGCGCGTGGACCAGAA
ATGCAAGTCCTCGGCCCAGATAGACCCGACTCCCGTGATCGTCACCTCCAAC
ACCAACATGTGCGCCGTGATTGACGGGAACTCAACGACCTTCGAACACCAGC
AGCCGTTGCAAGACCGGATGTTCAAATTTGAACTCACCCGCCGTCTGGATCA
TGACTTTGGGAAGGTCACCAAGCAGGAAGTCAAAGACTTTTTCCGGTGGGCA
AAGGATCACGTGGTTGAGGTGGAGCATGAATTCTACGTCAAAAAGGGTGGA
GCCAAGAAAAGACCCGCCCCCAGTGACGCAGATATAAGTGAGCCCAAACGG
GTGCGCGAGTCAGTTGCGCAGCCATCGACGTCAGACGCGGAAGCTTCGATC
AACTACGCAGACAGGTACCAAAACAAATGTTCTCGTCACGTGGGCATGAATC
TGATGCTGTTTCCCTGCAGACAATGCGAGAGAATGAATCAGAATTCAAATAT
CTGCTTCACTCACGGACAGAAAGACTGTTTAGAGTGCTTTCCCGTGTCAGAA
TCTCAACCCGTTTCTGTCGTCAAAAAGGCGTATCAGAAACTGTGCTACATTC
ATCATATCATGGGAAAGGTGCCAGACGCTTGCACTGCCTGCGATCTGGTCAA
TGTGGATTTGGATGACTGCATCTTTGAACAATAAATGATTTAAATCAGGTATG
GCTGCCGATGGTTATCTTCCAGATTGGCTCGAGGACAACCTCTCTGAGGGCATTC
GCGAGTGGTGGGACTTGAAACCTGGAGCCCCGAAGCCCAAAGCCAACCAGCAAA
AGCAGGACGACGGCCGGGGTCTGGTGCTTCCTGGCTACAAGTACCTCGGACCCTT
 
CAACGGACTCGACAAGGGGGAGCCCGTCAACGCGGCGGACGCAGCGGCCCTCGA
GCACGACAAGGCCTACGACCAGCAGCTCAAAGCGGGTGACAATCCGTACCTTCG
GTATAACCACGCCGACGCCGAGTTTCAGGAGCGTCTGCAAGAAGATACGTCTTTT
GGGGGCAACCTCGGGCGAGCAGTCTTCCAGGCCAAGAAGCGGGTTCTCGAACCT
CTCGGTCTGGTTGAGGAAGGCGCTAAGACGGCTCCTGGAAAGAAACGTCCGGTA
GAGCAGTCGCCACAAGAGCCAGACTCCTCCTCGGGCATCGGCAAGACAGGCCAG
CAGCCCGCTAAAAAGAGACTCAATTTTGGTCAGACTGGCGACTCAGAGTCAGTCC
CCGACCCACAACCTCTCGGAGAACCTCCAGCAACCCCCGCTGCTGTGGGACCTAC
TACAATGGCTTCAGGCGGTGGCGCACCAATGGCAGACAATAACGAAGGCGCCGA
CGGAGTGGGTAATGCCTCAGGAAATTGGCATTGCGATTCCACATGGCTGGGCGAC
AGAGTCATCACCACCAGCACCCGCACCTGGGCCTTGCCCACCTACAATAACCACC
TCTACAAGCAAATCTCCAGTGCTTCAACGGGGGCCAGCAACGACAACCACTACTT
CGGCTACAGCACCCCCTGGGGGTATTTTGATTTCAACAGATTCCACTGCCACTTTT
CACCACGTGACTGGCAGCGACTCATCAACAACAATTGGGGATTCCGGCCCAAGA
GACTCAACTTCAAACTCTTCAACATCCAAGTCAAGGAGGTCACGGCGAATGATGG
CGTCACAACCATCGCTAATAACCTTACCAGCACGGTTCAAGTCTTCTCGGACTCG
GAGTACCAGCTTCCGTACGTCCTCGGCTCTGCGCACCAGGGCTGCCTCCCTCCGTT
CCCGGCGGACGTGTTCATGATTCCGCAATACGGCTACCTGACGCTCAACAATGGC
AGCCAAGCCGTGGGACGTTCATCCTTTTACTGCCTGGAATATTTCCCTTCTCAGAT
GCTGAGAACGGGCAACAACTTTACCTTCAGCTACACCTTTGAGGAAGTGCCTTTC
CACAGCAGCTACGCGCACAGCCAGAGCCTGGACCGGCTGATGAATCCTCTCATCG
ACCAATACCTGTATTACCTGAACAGAACTCAAAATCCGTCCGGAAGTGCCCAAAA
CAAGGACTTGCTGTTTAGCCGTGGGTCTCCAGCTGGCATGTCTGTTCAGCCCAAA
 
AACTGGCTACCTGGACCCTGTTATCGGCAGCAGCGCGTTTCTAAAACAAAAACAG
ACAACAACAACAGCAATTTTACCTGGACTGGTGCTTCAAAATATAACCTCAATGG
GCGTGAATCCATCATCAACCCTGGCACTGCTATGGCCTCACACAAAGACGACGAA
GACAAGTTCTTTCCCATGAGCGGTGTCATGATTTTTGGAAAAGAGAGCGCCGGAG
CTTCAAACACTGCATTGGACAATGTCATGATTACAGACGAAGAGGAAATTAAAG
CCACTAACCCTGTGGCCACCGAAAGATTTGGGACCGTGGCAGTCAATTTCCAGAG
CAGCAGCACAGACCCTGCGACCGGAGATGTGCATGCTATGGGAGCATTACCTGG
CATGGTGTGGCAAGATAGAGACGTGTACCTGCAGGGTCCCATTTGGGCCAAAATT
CCTCACACAGATGGACACTTTCACCCGTCTCCTCTTATGGGCGGCTTTGGACTCAA
GAACCCGCCTCCTCAGATCCTCATCAAAAACACGCCTGTTCCTGCGAATCCTCCG
GCGGAGTTTTCAGCTACAAAGTTTGCTTCATTCATCACCCAATACTCCACAGGAC
AAGTGAGTGTGGAAATTGAATGGGAGCTGCAGAAAGAAAACAGCAAGCGCTGGA
ATCCCGAAGTGCAGTACACATCCAATTATGCAAAATCTGCCAACGTTGATTTTAC
TGTGGACAACAATGGACTTTATACTGAGCCTCGCCCCATTGGCACCCGTTACCTT
ACCCGTCCCCTGTAATTACGTGTTAATCAATAAACCGGTTAATTCGTGTCAGTTGA
ACTTTGGTCTCATGTCGTTATTATCTTATCTGGTCACCAGATACGTAGATAAGTAG
CATGGCGGGTTAATCATTAACTACAGCCCGGGCGTTTAAACAGCGGGCGGAGGG
GTGGAGTCGTGACGTGAATTACGTCATAGGGTTAGGGAGGTCCTGTATTAGAGGT
CACGTGAGTGTTTTGCGACATTTTGCGACACCATGTGGTCACGCTGGGGGGGGG
GGCCCGAGTGAGCACGCAGGGTCTCCATTTTGAAGCGGGAGGTTTGAACGAGCGCT
GGCGCGCTCACTGGCCGTCGTTTTACAACGTCGTGACTGGGAAAACCCTGGCGTT
ACCCAACTTAATCGCCTTGCAGCACATCCCCCTTTCGCCAGCTGGCGTAATAGCG
AAGAGGCCCGCACCGATCGCCCTTCCCAACAGTTGCGCAGCCTGAATGGCGAATG
 
GGTAAATTGTAAACGTTAATATTTTGTTAAAATTCGCGTTAAATTTTTGTTAAATC
AGCTCATTTTTTAACCAATAGGCCGAAATCGGCAAAATCCCTTATAAATCAAAAG
AATAGACCGAGATAGGGTTGAGTGTTGTTCCAGTTTGGAACAAGAGTCCACTATT
AAAGAACGTGGACTCCAACGTCAAAGGGCGAAAAACCGTCTATCAGGGCGATGG
CCCACTACGTGAACCATCACCCTAATCAAGTTTTTTGGGGTCGAGGTGCCGTAAA
GCACTAAATCGGAACCCTAAAGGGAGCCCCCGATTTAGAGCTTGACGGGGAAAG
CCGGCGAACGTGGCGAGAAAGGAAGGGAAGAAAGCGAAAGGAGCGGGCGCTAG
GGCGCTGGCAAGTGTAGCGGTCACGCTGCGCGTAACCACCACACCCGCCGCGCTT
AATGCGCCGCTACAGGGCGCGTGGTGGCACTTTTCGGGGAAATGTGCGCGGAAC
CCCTATTTGTTTATTTTTCTAAATACATTCAAATATGTATCCGCTCATGAGACAAT
AACCCTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTCACCCA
GAAACGCTGGTGAAAGTAAAAGATGCTGAAGATCAGTTGGGTGCACGAGTGGGTTACA
TCGAACTGGATCTCAACAGCGGTAAGATCCTTGAGAGTTTTCGCCCCGAAGAACGTTTT
CCAATGATGAGCACTTTTAAAGTTCTGCTATGTGGCGCGGTATTATCCCGTATTGACGC
CGGGCAAGAGCAACTCGGTCGCCGCATACACTATTCTCAGAATGACTTGGTTGAGTAC
TCACCAGTCACAGAAAAGCATCTTACGGATGGCATGACAGTAAGAGAATTATGCAGTGC
TGCCATAAGCATGAGTGATAACACTGCGGCCAACTTACTTCTGACAACGATCGGAGGA
CCGAAGGAGCTAACCGCTTTTTTTCACAACATGGGGGATCATGTAACTCGCCTTGATCG
TTGGGAACCGGAGCTGAATGAAGCCATACCAAACGACGAGCGTGACACCACGATGCCT
GTAGCAATGGCAACAACGTTGCGCAAACTATTAACTGGCGAACTACTTACTCTAGCTTC
CCGGCAACAATTAATAGACTGGATGGAGGCGGATAAAGTTGCAGGACCACTTCTGCGC
TCGGCCCTTCCGGCTGGCTGGTTTATTGCTGATAAATCTGGAGCCGGTGAGCGTGGGT
 
CTCGCGGTATCATTGCAGCACTGGGGCCAGATGGTAAGCCCTCCCGTATCGTAGTTAT
CTACACGACGGGCAGTCAGGCAACTATGGATGAACGAAATAGACAGATCGCTGAGATA
GGTGCCTCACTGATTAAGCATTGGTAACTGTCAGACCAAGTTTACTCATATATACTTT
AGATTGATTTAAAACTTCATTTTTAATTTAAAAGGATCTAGGTGAAGATCCTTTTT
GATAATCTCATGACCAAAATCCCTTAACGTGAGTTTTCGTTCCACTGAGCGTCAG
ACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACC
AAATACTGTCCTTCTAGTGTAGCCGTAGTTAGGCCACCACTTCAAGAACTCTGTA
GCACCGCCTACATACCTCGCTCTGCTAATCCTGTTACCAGTGGCTGCTGCCAGTGG
CGATAAGTCGTGTCTTACCGGGTTGGACTCAAGACGATAGTTACCGGATAAGGCG
CAGCGGTCGGGCTGAACGGGGGGTTCGTGCACACAGCCCAGCTTGGAGCGAACG
ACCTACACCGAACTGAGATACCTACAGCGTGAGCATTGAGAAAGCGCCACGCTTC
CCGAAGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCCTGTCG
GGTTTCGCCACCTCTGACTTGAGCGTCGATTTTTGTGATGCTCGTCAGGGGGGCCG
AGCCTATGGAAAAACGCCAGCAACGCGGCCTTTTTACGGTTCCTGGCCTTTTGCT
GGCCTTTTGCTCACATGTTCTTTCCTGCGTTATCCCCTGATTCTGTGGATAACCGT
ATTACCGCCTTTGAGTGAGCTGATACCGCTCGCCGCAGCCGAACGACCGAGCGCA
GCGAGTCAGTGAGCGAGGAAGCGGAAGAGCGCCCAATACGCAAACCGCCTCTCC
CCGCGCGTTGGCCGATTCATTAATGCAGCTGGCACGACAGGTTTCCCGACTGGAA
AGCGGGCAGTGAGCGCAACGCAATTAATGTGAGTTACCTCACTCATTAGGCACCC
 
CAGGCTTTACACTTTATGCTTCCGGCTCCTATGTTGTGTGGAATTGTGAGCGGATA
ACAATTTCACACAGGAAACAGCTATGACCATGATTACGCCAA
**<AAV1 #3-32 variant>**
**AAV-2 Rep gene** #3-65 Cap gene *Ampicillin Resistance (bla) gene(SEQ* ID NO: 7) 
GCGCGCCGATATCGTTAACGCCCCGCGCCGGCCGCTCTAGAACTAGTGGA
TCCCCCGGAAGATCAGAAGTTCCTATTCCGAAGTTCCTATTCTCTAGAAAGTATA
GGAACTTCTGATCTGCGCAGCCGCCATGCCGGGGTTTTACGAGATTGTGATTA
AGGTCCCCAGCGACCTTGACGGGCATCTGCCCGGCATTTCTGACAGCTTTGT
GAACTGGGTGGCCGAGAAGGAATGGGAGTTGCCGCCAGATTCTGACATGGA
TCTGAATCTGATTGAGCAGGCACCCCTGACCGTGGCCGAGAAGCTGCAGCG
CGACTTTCTGACGGAATGGCGCCGTGTGAGTAAGGCCCCGGAGGCCCTTTT
CTTTGTGCAATTTGAGAAGGGAGAGAGCTACTTCCACATGCACGTGCTCGTG
GAAACCACCGGGGTGAAATCCATGGTTTTGGGACGTTTCCTGAGTCAGATTC
GCGAAAAACTGATTCAGAGAATTTACCGCGGGATCGAGCCGACTTTGCCAAA
CTGGTTCGCGGTCACAAAGACCAGAAATGGCGCCGGAGGCGGGAACAAGGT
GGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATC
TCACGGAGCGTAAACGGTTGGTGGCGCAGCATCTGACGCACGTGTCGCAGA
CGCAGGAGCAGAACAAAGAGAATCAGAATCCCAATTCTGATGCGCCGGTGA
TCAGATCAAAAACTTCAGCCAGGTACATGGAGCTGGTCGGGTGGCTCGTGG
ACAAGGGGATTACCTCGGAGAAGCAGTGGATCCAGGAGGACCAGGCCTCAT
ACATCTCCTTCAATGCGGCCTCCAACTCGCGGTCCCAAATCAAGGCTGCCTT
GGACAATGCGGGAAAGATTATGAGCCTGACTAAAACCGCCCCCGACTACCT
 
GGTGGGCCAGCAGCCCGTGGAGGACATTTCCAGCAATCGGATTTATAAAATT
TTGGAACTAAACGGGTACGATCCCCAATATGCGGCTTCCGTCTTTCTGGGAT
GGGCCACGAAAAAGTTCGGCAAGAGGAACACCATCTGGCTGTTTGGGCCTG
CAACTACCGGGAAGACCAACATCGCGGAGGCCATAGCCCACACTGTGCCCT
TCTACGGGTGCGTAAACTGGACCAATGAGAACTTTCCCTTCAACGACTGTGT
CGACAAGATGGTGATCTGGTGGGAGGAGGGGAAGATGACCGCCAAGGTCGT
GGAGTCGGCCAAAGCCATTCTCGGAGGAAGCAAGGTGCGCGTGGACCAGAA
ATGCAAGTCCTCGGCCCAGATAGACCCGACTCCCGTGATCGTCACCTCCAAC
ACCAACATGTGCGCCGTGATTGACGGGAACTCAACGACCTTCGAACACCAGC
AGCCGTTGCAAGACCGGATGTTCAAATTTGAACTCACCCGCCGTCTGGATCA
TGACTTTGGGAAGGTCACCAAGCAGGAAGTCAAAGACTTTTTCCGGTGGGCA
AAGGATCACGTGGTTGAGGTGGAGCATGAATTCTACGTCAAAAAGGGTGGA
GCCAAGAAAAGACCCGCCCCCAGTGACGCAGATATAAGTGAGCCCAAACGG
GTGCGCGAGTCAGTTGCGCAGCCATCGACGTCAGACGCGGAAGCTTCGATC
AACTACGCAGACAGGTACCAAAACAAATGTTCTCGTCACGTGGGCATGAATC
TGATGCTGTTTCCCTGCAGACAATGCGAGAGAATGAATCAGAATTCAAATAT
CTGCTTCACTCACGGACAGAAAGACTGTTTAGAGTGCTTTCCCGTGTCAGAA
TCTCAACCCGTTTCTGTCGTCAAAAAGGCGTATCAGAAACTGTGCTACATTC
ATCATATCATGGGAAAGGTGCCAGACGCTTGCACTGCCTGCGATCTGGTCAA
TGTGGATTTGGATGACTGCATCTTTGAACAATAAATGATTTAAATCAGGTATG
GCTGCCGATGGTTATCTTCCAGATTGGCTCGAGGACAACCTCTCTGAGGGCATTC
GCGAGTGGTGGGACTTGAAACCTGGAGCCCCGAAGCCCAAAGCCAACCAGCAAA
AGCAGGACGACGGCCGGGGTCTGGTGCTTCCTGGCTACAAGTACCTCGGACCCTT
 
CAACGGACTCGACAAGGGGGAGCCCGTCAACGCGGCGGACGCAGCGGCCCTCGA
GCACGACAAGGCCTACGACCAGCAGCTCAAAGCGGGTGACAATCCGTACCTTCG
GTATAACCACGCCGACGCCGAGTTTCAGGAGCGTCTGCAAGAAGATACGTCTTTT
GGGGGCAACCTCGGGCGAGCAGTCTTCCAGGCCAAGAAGCGGGTTCTCGAACCT
CTCGGTCTGGTTGAGGAAGGCGCTAAGACGGCTCCTGGAAAGAAACGTCCGGTA
GAGCAGTCGCCACAAGAGCCAGACTCCTCCTCGGGCATCGGCAAGACAGGCCAG
CAGCCCGCTAAAAAGAGACTCAATTTTGGTCAGACTGGCGACTCAGAGTCAGTCC
CCGACCCACAACCTCTCGGAGAACCTCCAGCAACCCCCGCTGCTGTGGGACCTAC
TACAATGGCTTCAGGCGGTGGCGCACCAATGGCAGACAATAACGAAGGCGCCGA
CGGAGTGGGTAATGCCTCAGGAAATTGGCATTGCGATTCCACATGGCTGGGCGAC
AGAGTCATCACCACCAGCACCCGCACCTGGGCCTTGCCCACCTACAATAACCACC
TCTACAAGCAAATCTCCAGTGCTTCAACGGGGGCCAGCAACGACAACCACTACTT
CGGCTACAGCACCCCCTGGGGGTATTTTGATTTCAACAGATTCCACTGCCACTTTT
CACCACGTGACTGGCAGCGACTCATCAACAACAATTGGGGATTCCGGCCCAAGA
GACTCAACTTCAAACTCTTCAACATCCAAGTCAAGGAGGTCACGACGAATGATGG
CGTCACAACCATCGCTAATAACCTTACCAGCACGGTTCAAGTCTTCTCGGACTCG
GAGTACCAGCTTCCGTACGTCCTCGGCTCTGCGCACCAGGGCTGCCTCCCTCCGTT
CCCGGCGGACGTGTTCATGATTCCGCAATACGGCTACCTGACGCTCAACAATGGC
AGCCAAGCCGTGGGACGTTCATCCTTTTACTGCCTGGAATATTTCCCTTCTCAGAT
GCTGAGAACGGGCAACAACTTTACCTTCAGCTACACCTTTGAGGAAGTGCCTTTC
CACAGCAGCTACGCGCACAGCCAGAGCCTGGACCGGCTGATGAATCCTCTCATCG
ACCAATACCTGTATTACCTGAACAGAACTCAAAATCAGTCCGGAAGTACCCAAAA
CAAGGACTTGCTGTTTAGCCGTGGGTCTCCAGCTGGCATGTCTGTTCAGCCCAAA
 
AACTGGCTACCTGGACCCTGTTATCGGCAGCAGCGCGTTTCTAAAACAAAAACAG
ACAACAACAACAGCAATTTTACCTGGACTGGTGCTTCAAAATATAACCTCAATGG
GCGTGAATCCATCATCAACCCTGGCACTGCTATGGCCTCACACAAAGACGACGAA
GACAAGTTCTTTCCCATGAGCGGTGTCATGATTTTTGGAAAAGAGAGCGCCGGAG
CTTCAAACACTGCATTGGACAATGTCATGATTACAGACGAAGAGGAAATTAAAG
CCACTAACCCTGTGGCCACCGAAAGATTTGGGACCGTGGCAGTCAATTTCCAGAG
CAGCAGCACAGACCCTGCGACCGGAGATGTGCATGCTATGGGAGCATTACCTGG
CATGGTGTGGCAAGATAGAGACGTGTACCTGCAGGGTCCCATTTGGGCCAAAATT
CCTCACACAGATGGACACTTTCACCCGTCTCCTCTTATGGGCGGCTTTGGACTCAA
GAACCCGCCTCCTCAGATCCTCATCAAAAACACGCCTGTTCCTGCGAATCCTCCG
GCGGAGTTTTCAGCTACAAAGTTTGCTTCATTCATCACCCAATACTCCACAGGAC
AAGTGAGTGTGGAAATTGAATGGGAGCTGCAGAAAGAAAACAGCAAGCGCTGGA
ATCCCGAAGTGCAGTACACATCCAATTATGCAAAATCTGCCAACGTTGATTTTAC
TGTGGACAACAATGGACTTTATACTGAGCCTCGCCCCATTGGCACCCGTTACCTT
ACCCGTCCCCTGTAATTACGTGTTAATCAATAAACCGGTTAATTCGTGTCAGTTGA
ACTTTGGTCTCATGTCGTTATTATCTTATCTGGTCACCAGATACGTAGATAAGTAG
CATGGCGGGTTAATCATTAACTACAGCCCGGGCGTTTAAACAGCGGGCGGAGGG
GTGGAGTCGTGACGTGAATTACGTCATAGGGTTAGGGAGGTCCTGTATTAGAGGT
CACGTGAGTGTTTTGCGACATTTTGCGACACCATGTGGTCACGCTGGGGGGGGG
GGCCCGAGTGAGCACGCAGGGTCTCCATTTTGAAGCGGGAGGTTTGAACGAGCGCT
GGCGCGCTCACTGGCCGTCGTTTTACAACGTCGTGACTGGGAAAACCCTGGCGTT
ACCCAACTTAATCGCCTTGCAGCACATCCCCCTTTCGCCAGCTGGCGTAATAGCG
AAGAGGCCCGCACCGATCGCCCTTCCCAACAGTTGCGCAGCCTGAATGGCGAATG
 
GGTAAATTGTAAACGTTAATATTTTGTTAAAATTCGCGTTAAATTTTTGTTAAATC
AGCTCATTTTTTAACCAATAGGCCGAAATCGGCAAAATCCCTTATAAATCAAAAG
AATAGACCGAGATAGGGTTGAGTGTTGTTCCAGTTTGGAACAAGAGTCCACTATT
AAAGAACGTGGACTCCAACGTCAAAGGGCGAAAAACCGTCTATCAGGGCGATGG
CCCACTACGTGAACCATCACCCTAATCAAGTTTTTTGGGGTCGAGGTGCCGTAAA
GCACTAAATCGGAACCCTAAAGGGAGCCCCCGATTTAGAGCTTGACGGGGAAAG
CCGGCGAACGTGGCGAGAAAGGAAGGGAAGAAAGCGAAAGGAGCGGGCGCTAG
GGCGCTGGCAAGTGTAGCGGTCACGCTGCGCGTAACCACCACACCCGCCGCGCTT
AATGCGCCGCTACAGGGCGCGTGGTGGCACTTTTCGGGGAAATGTGCGCGGAAC
CCCTATTTGTTTATTTTTCTAAATACATTCAAATATGTATCCGCTCATGAGACAAT
AACCCTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTCACCCA
GAAACGCTGGTGAAAGTAAAAGATGCTGAAGATCAGTTGGGTGCACGAGTGGGTTACA
TCGAACTGGATCTCAACAGCGGTAAGATCCTTGAGAGTTTTCGCCCCGAAGAACGTTTT
CCAATGATGAGCACTTTTAAAGTTCTGCTATGTGGCGCGGTATTATCCCGTATTGACGC
CGGGCAAGAGCAACTCGGTCGCCGCATACACTATTCTCAGAATGACTTGGTTGAGTAC
TCACCAGTCACAGAAAAGCATCTTACGGATGGCATGACAGTAAGAGAATTATGCAGTGC
TGCCATAAGCATGAGTGATAACACTGCGGCCAACTTACTTCTGACAACGATCGGAGGA
CCGAAGGAGCTAACCGCTTTTTTTCACAACATGGGGGATCATGTAACTCGCCTTGATCG
TTGGGAACCGGAGCTGAATGAAGCCATACCAAACGACGAGCGTGACACCACGATGCCT
GTAGCAATGGCAACAACGTTGCGCAAACTATTAACTGGCGAACTACTTACTCTAGCTTC
CCGGCAACAATTAATAGACTGGATGGAGGCGGATAAAGTTGCAGGACCACTTCTGCGC
TCGGCCCTTCCGGCTGGCTGGTTTATTGCTGATAAATCTGGAGCCGGTGAGCGTGGGT
 
CTCGCGGTATCATTGCAGCACTGGGGCCAGATGGTAAGCCCTCCCGTATCGTAGTTAT
CTACACGACGGGCAGTCAGGCAACTATGGATGAACGAAATAGACAGATCGCTGAGATA
GGTGCCTCACTGATTAAGCATTGGTAACTGTCAGACCAAGTTTACTCATATATACTTT
AGATTGATTTAAAACTTCATTTTTAATTTAAAAGGATCTAGGTGAAGATCCTTTTT
GATAATCTCATGACCAAAATCCCTTAACGTGAGTTTTCGTTCCACTGAGCGTCAG
ACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATC
AAGAGCTACCAACTCTTTTTCCGAAGGTAACTGGCTTCAGCAGAGCGCAGATACC
AAATACTGTCCTTCTAGTGTAGCCGTAGTTAGGCCACCACTTCAAGAACTCTGTA
GCACCGCCTACATACCTCGCTCTGCTAATCCTGTTACCAGTGGCTGCTGCCAGTGG
CGATAAGTCGTGTCTTACCGGGTTGGACTCAAGACGATAGTTACCGGATAAGGCG
CAGCGGTCGGGCTGAACGGGGGGTTCGTGCACACAGCCCAGCTTGGAGCGAACG
ACCTACACCGAACTGAGATACCTACAGCGTGAGCATTGAGAAAGCGCCACGCTTC
CCGAAGGGAGAAAGGCGGACAGGTATCCGGTAAGCGGCAGGGTCGGAACAGGA
GAGCGCACGAGGGAGCTTCCAGGGGGAAACGCCTGGTATCTTTATAGTCCTGTCG
GGTTTCGCCACCTCTGACTTGAGCGTCGATTTTTGTGATGCTCGTCAGGGGGGCCG
AGCCTATGGAAAAACGCCAGCAACGCGGCCTTTTTACGGTTCCTGGCCTTTTGCT
GGCCTTTTGCTCACATGTTCTTTCCTGCGTTATCCCCTGATTCTGTGGATAACCGT
ATTACCGCCTTTGAGTGAGCTGATACCGCTCGCCGCAGCCGAACGACCGAGCGCA
GCGAGTCAGTGAGCGAGGAAGCGGAAGAGCGCCCAATACGCAAACCGCCTCTCC
CCGCGCGTTGGCCGATTCATTAATGCAGCTGGCACGACAGGTTTCCCGACTGGAA
AGCGGGCAGTGAGCGCAACGCAATTAATGTGAGTTACCTCACTCATTAGGCACCC
 
CAGGCTTTACACTTTATGCTTCCGGCTCCTATGTTGTGTGGAATTGTGAGCGGATA
ACAATTTCACACAGGAAACAGCTATGACCATGATTACGCCAA
**<AAV1 #3-65 variant>**

### Construction of wild-type AAV1

A calcium-phosphate complex formed of 17 µg of a packaging plasmid containing the *rep* gene of AAV2 and the *cap* gene of AAV1, 17 µg of an ITR flanked reporter gene (pCMV-GFP, pCMV-LacZ, or pCMV-FGF12-IRES-GFP), and 17 µg of pHelper was transfected into AAV293 cells, and after approximately 48 hours, only a cell pellet was collected to extract intracellular AAV through freezing-thawing. Afterward, cell debris was removed through centrifugation, and 10 U/mL of benzonase was incubated at 37 °C for 30 minutes to remove a nucleic acid derived from virus-producing cells.

### 3) Purification of AAV1 #3-32 and #3-65 variants

An AAV solution was subjected to ultracentrifugation using an iodixanol gradient. 15%, 25%, 40%, and 54% iodixanol solutions were prepared and loaded sequentially in ultracentrifuge tubes, and the AAV solution was loaded on top of them. After tube sealing, ultracentrifugation was performed (42,000 RPM, 18 °C, 2 hrs) using an Optima XE-90 Ultracentrifuge (BECKMAN COULTER) and a Vti65.2 rotor. The AAV layer located between the 54% and 40% iodixanol gradients was extracted, and buffer exchange was performed with PBS buffer containing 0.01% Tween 20 using an Amicon^{®} Ultra-15 Centrifugal Filter (MWCO 100,000).

### 4) Measurement of titers of AAV1 #3-32 and #3-65 variants

After extracting a viral genome by treating a virus having resistance to DNase I (5U) with protease K, the titers of the wild-type AAV1 and the AAV1 #3-32 and #3-65 variants, carrying CMV-FGF12-IRES-GFP, were quantified with the standards thereof by qPCR using quantitative PCR (qPCR) primers for CMV (5-ATGGTGATGCGGTTTTGGCAG-3: SEQ ID NO: 10 and 5-GGCGGAGTTGTTACGACATTTTGG-3: SEQ ID NO: 11).

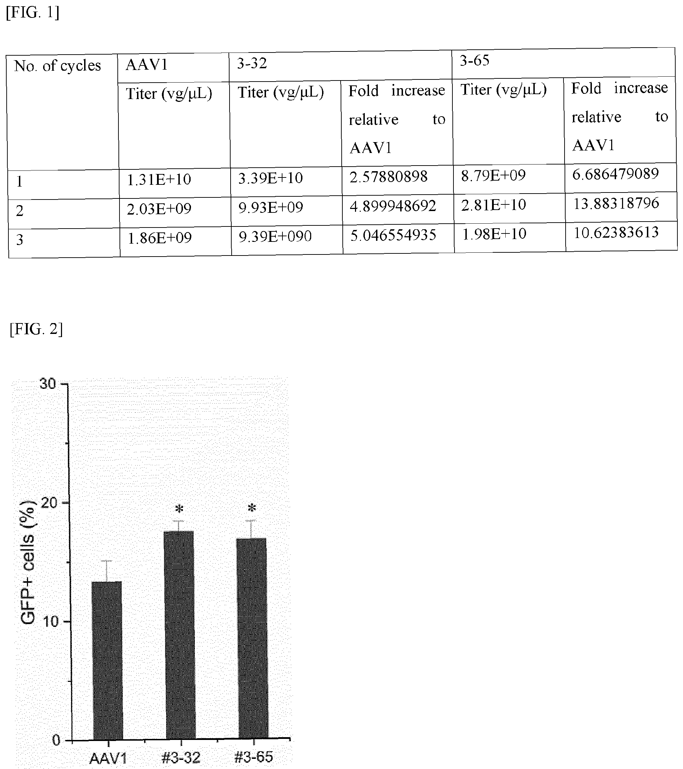

The packaging efficiency of the wild-type AAV1 and the recombinant AAV1 vectors (#3-32 and #3-65 variants) was compared by genomic titer, and the results are shown in FIG. 1. These results show that the packaging efficiency of the #3-32 and #3-65 variants is higher than that of the wild-type AAV1, indicating the potential to be maintained as evolutionarily superior individuals.

### Example 2: Confirmation of recombinant AAV1 variant infection

### 1) In vitro experiment

For analysis of gene delivery efficiency and location as a reporter gene, the wild-type AAV1 and the #3-32 and #3-65 variants in which CMV-GFP was loaded were packaged and used to infect HEK293T (2x10⁴ cells/20 µL).

CMV-FGF12-IRES-GFP was loaded in each virus, and when GFP was loaded, and after 48 hours of HEK293T infection (MOI 10,000), the ratio (%) of GFP-expressing cells among the total cultured cells was analyzed through flow cytometry to confirm infection ability. The results are shown in FIG. 2.

FIG. 2 shows the analysis of the improvement in HEK293T transduction efficiency to by the proportion of cells expressing GFP among the total cultured cells.

### 2) In vivo experiment

Each of the AAV1 wild-type and the #3-32 and #3-65 variants, which carry CMV-LacZ, was packaged and injected intratracheally into 8-week-old C57BL/6 male mice in the form of an aerosol (1x10¹¹ vg/100 µL).

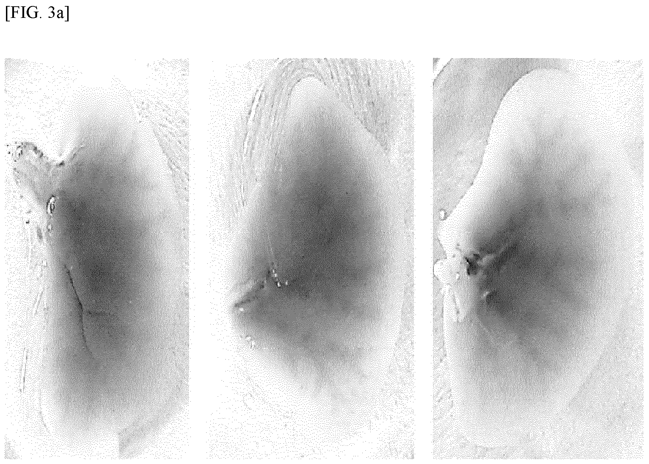

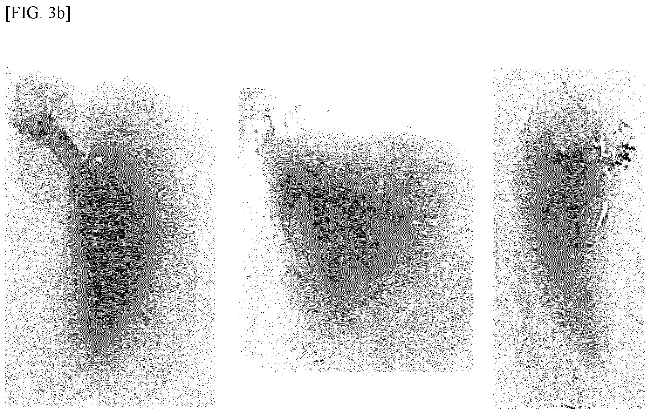

In order to confirm the pulmonary bronchial specificity of the recombinant AAV1 vectors (#3-32 and #3-65 variants), as a result of confirming LacZ expression by staining the lungs extracted from the 8-week-old C57BL/6 male mice with whole-mount β-galactosidase one week after injection, as shown in FIGS. 3A and 3B, it was confirmed that the recombinant AAV1 vectors (#3-32 and #3-65 variants) were effectively delivered to the pulmonary bronchi.

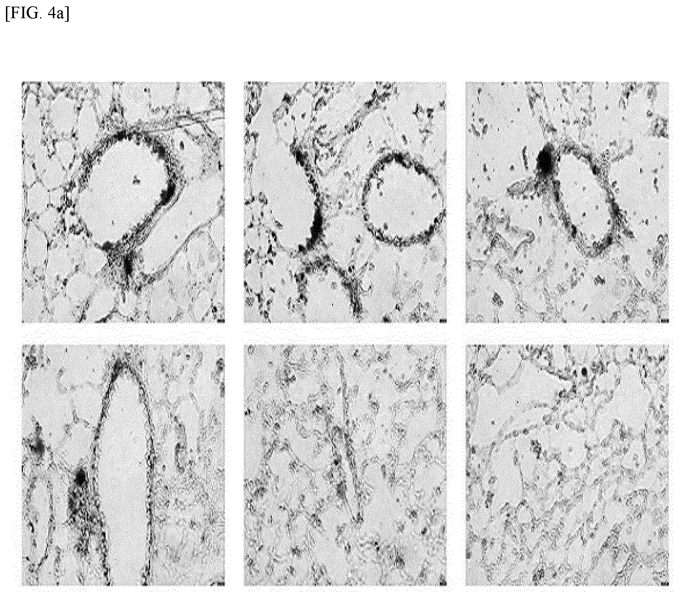

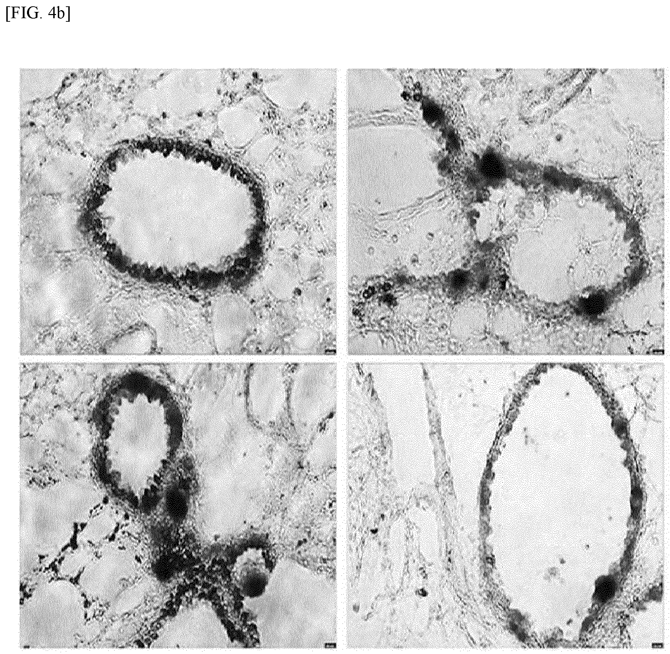

In addition, to confirm the pulmonary bronchial specificity of the recombinant AAV1 vectors (#3-32 and #3-65 variants), 8-week-old C57BL/6 male mice were injected, and lungs were extracted one week later, and LacZ expression in the pulmonary bronchi was confirmed through eosin / LacZ staining. As a result, as shown in FIGS. 4A and 4B, it was confirmed that the recombinant AAV1 vectors were specifically delivered to the pulmonary bronchi.

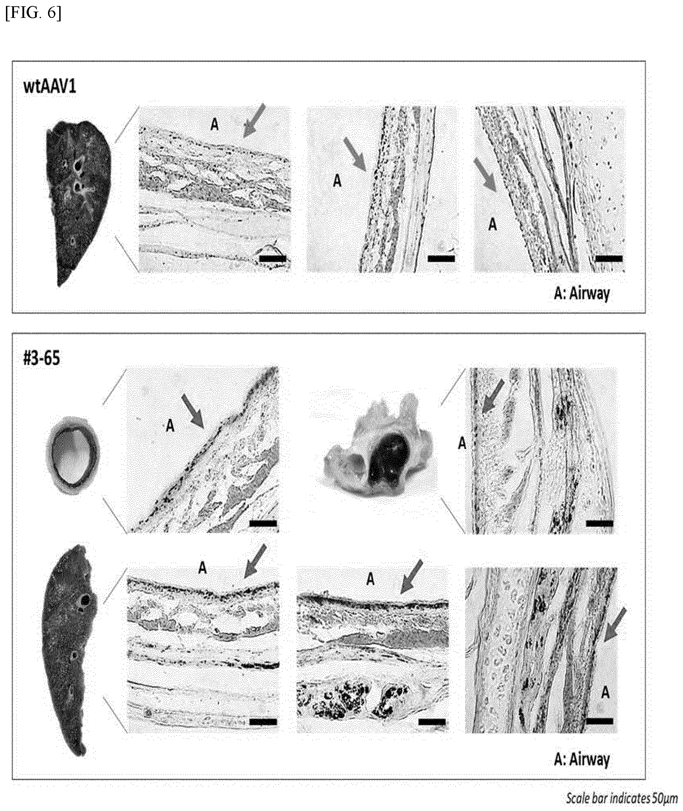

In addition, LacZ-loaded wtAAV1 (1.25x1011vg/kg) and #3-65 (5.38x1011vg/kg) vector were dispersed in PBS+0.01% Tween20 and then injected into the porcine airway in the form of 1 mL liquid, and then the test animals were sacrificed after two weeks and subjected to 4% PFA fixation and X-gal staining, thereby confirming that compared to wtAAV1, which shows no gene expression in the lung section with the airway, #3-65 shows the local expression of LacZ in the airway in the airway area and lung tissue (FIG. 6).

## Claims

1. A mutant of the AAV1 capsid protein in which an amino acid present at one or more of positions 326, 452, and 456 in the amino acid sequence of the wild-type AAV1 capsid protein, represented by SEQ ID NO: 1, is substituted.

2. The mutant of claim 1, wherein, in the amino acid sequence of the wild type AAV1 capsid protein represented by SEQ ID NO: 1, threonine at position 326 is substituted with alanine, and glutamine at position 452 is substituted with proline.

3. The mutant of claim 1, wherein, in the wild type AAV1 capsid protein represented by SEQ ID NO: 1, alanine at position 456 is substituted with threonine.

4. A nucleic acid encoding the mutant of the AAV1 capsid protein of claim 2.

5. A nucleic acid encoding the mutant of the AAV1 capsid protein of claim 3.

6. The nucleic acid of claim 4, which has a base sequence represented by SEQ ID NO: 4.

7. The nucleic acid of claim 5, which has a base sequence represented by SEQ ID NO: 5.

8. A recombinant AAV1 vector comprising the nucleic acid encoding the mutant of the AAV1 capsid protein of claim 4 or 5.

9. The recombinant AAV1 vector of claim 8, which has an improved transduction profile for the pulmonary bronchi as compared to a wild-type AAV1 virus vector.

10. A pharmaceutical composition comprising the recombinant AAV1 vector of claim 8.

11. The pharmaceutical composition of claim 10, further comprising:
a pharmaceutically acceptable carrier.

12. The pharmaceutical composition of claim 10, which is for preventing or treating bronchitis or bronchiectasis.

13. A gene delivery vehicle comprising the recombinant AAV1 vector of claim 8.
